Europäisches Patentamt

European Patent Office

Office européen des brevets

(19)

(11) **EP 1 051 148 B1**

(12) **FASCICULE DE BREVET EUROPEEN**

(45) Date de publication et mention
de la délivrance du brevet:
**30.06.2004 Bulletin 2004/27**

(21) Numéro de dépôt: **99956292.9**

(22) Date de dépôt: **06.12.1999**

(51) Int Cl.⁷: $A61K\ 7/46$

(86) Numéro de dépôt international:
**PCT/IB1999/001948**

(87) Numéro de publication internationale:
**WO 2000/033804 (15.06.2000 Gazette 2000/24)**

(54) **COMPOSITION PARFUMANTE TRANSPARENTE**

DURCHSICHTIGE RIECHSTOFF-ZUSAMMENSETZUNG

TRANSPARENT PERFUME COMPOSITION

(84) Etats contractants désignés:
**AT BE CH CY DE DK ES FI FR GB GR IE IT LI LU
MC NL PT SE**

(30) Priorité: **08.12.1998 CH 243798**

(43) Date de publication de la demande:
**15.11.2000 Bulletin 2000/46**

(73) Titulaire: **Firmenich S.A.
1211 Geneva 8 (CH)**

(72) Inventeur: **STORA, Thierry
F-01630 Sergy (FR)**

(74) Mandataire:
**Salvaterra-Garcia, Maria de Lurdes et al
Firmenich SA
Département des Brevets
Case Postale 239
1211 Genève 8 (CH)**

(56) Documents cités:
**WO-A-97/06777          WO-A-99/06473
FR-A- 2 618 351          GB-A- 2 283 914
US-A- 4 948 578          US-A- 5 534 246
US-B- 4 948 578**

• **DATABASE WPI Derwent Publications Ltd.,
London, GB; AN 1999-161036 XP002133373 & JP
11 018709 A (FUJI SEIYU), 26 janvier 1999
(1999-01-26)**

## Description

### Domaine technique

La présente invention a trait au domaine de la parfumerie. Elle concerne, plus particulièrement, un parfum, une eau de toilette ou une eau de Cologne transparents et essentiellement dépourvus de solvants volatiles organiques courants.

### Technique antérieure

Dans la préparation des parfums, eaux de toilette et eaux de Cologne l'utilisation d'éthanol en tant que solvant est encore très répandue. L'éthanol permet de bien solubiliser les ingrédients parfumants auxquels le parfumeur a eu recours. On arrive ainsi facilement à incorporer chaque ingrédient à la concentration souhaitée et à obtenir une solution transparente. Pour de telles raisons, la plupart des parfums et eaux de toilette que l'on trouve sur le marché contiennent encore de l'éthanol, généralement entre 50 et 95% par volume.

Actuellement, on assiste à une préférence du consommateur pour des parfums sans alcool, ou avec un contenu réduit en alcool. Ainsi, on cherche à remplacer l'éthanol dans les produits susmentionnés.

Il serait souhaitable de remplacer l'éthanol par de l'eau, ou par un solvant organique qui ne laisse pas de résidus importants sur la peau, ou encore par un mélange de l'un ou plusieurs de ces solvants avec de l'eau.

Cependant, dans ce contexte, l'utilisation de l'eau ou d'un mélange de celle-ci avec un solvant organique non volatile conduit à des problèmes de solubilité des ingrédients parfumants, ces composés étant hydrophobes, dans la phase aqueuse. Bien que l'on connaisse des émulsions de type huile-dans-eau de ces ingrédients, ces émulsions ne sont en général pas transparentes.

Par ailleurs, on connaît de l'art antérieur des compositions parfumantes transparentes sous forme de microémulsions contenant des quantités importantes d'agents tensioactifs. Dans ces microémulsions, le parfum (la phase huileuse) se trouve sous forme de gouttes d'une taille d'environ 5-50 nm dispersées dans l'eau, et grâce à la petite taille de ces gouttes, le mélange, c'est-à-dire la microémulsion, est transparent. Cependant, une quantité importante d'agents tensioactifs par rapport à la quantité d'ingrédients parfumants à incorporer n'est pas souhaitable, et limite de façon considérable la quantité de parfum que l'on peut incorporer dans le mélange.

On connaît également un autre type de compositions transparentes, à savoir les nanoémulsions, qui sont caractérisées par une taille moyenne des gouttes de la phase huileuse inférieure à 30-75 nm environ. Cette taille est suffisamment petite pour que l'émulsion soit translucide ou partiellement transparente. Bien que ces émulsions aient l'avantage de comprendre de moins grandes quantités d'agents tensioactifs, elles présentent, néanmoins l'inconvénient d'un procédé de préparation souvent difficile et délicat. De telles émulsions font par exemple l'objet de la demande EP 728 460. Plus précisément, on décrit dans ce document une nanoémulsion huile-dans-eau "transparente" dans laquelle on ajoute un lipide amphiphile, non ionique et liquide, à une température inférieure à 45°C afin d'obtenir l'émulsion souhaitée. Ce lipide est utilisé dans des proportions allant de 2 à 10% en poids, par rapport au poids total de la phase lipidique. En tant que phase huileuse, la demande de brevet décrit l'utilisation, entre autres, d'huiles essentielles d'origine naturelle et synthétique. Les émulsions décrites dans ce brevet sont utilisées dans des compositions cosmétiques en raison de leurs propriétés de pénétration lors d'une application sur la peau.

Enfin, une émulsion "classique", dans laquelle la taille moyenne des gouttes dispersées est de plus de 100 nm, est décrite dans le brevet US 5,798,111. Ce brevet a pour objet une émulsion cosmétique à haute viscosité et plus particulièrement un gel semi-solide, transparent, comprenant de 10 à 97% en poids d'une phase aqueuse contenant du 2-méthyl-1,3-propanediol, et de 2 à 90% en poids d'une phase huileuse qui comprend une huile de silicone. Les gels de ce type ont une turbidité optique inférieure à 50 NTU à 21°C. En sus des composés cités ci-dessus, ces compositions cosmétiques peuvent contenir des agents tensioactifs non ioniques. Les compositions décrites dans ce document sont trop visqueuses pour être utilisées dans des compositions parfumantes telles que des parfums, eaux de toilette ou eaux de Cologne.

Le document brevet FR-A-2 618 351 décrit un parfum en crème sous forme d'une émulsion huile-dans-eau, l'huile contenant au moins un ingrédient parfumant, ladite composition comprenant de 50 à 80 % en poids d'une phase continue, les indices de réfraction de ladite phase dispersée et de ladite phase continue étant égaux. Pour une crême, la viscosité attendue de l'émulsion est supérieure à 10 Pa. s.

### Exposé de l'invention

La présente invention a pour but de résoudre les différents problèmes rencontrés dans l'art antérieur au moyen d'une composition parfumante essentiellement dépourvue de solvants organiques volatiles ou VOC définis par l'E.P.A. (Environmental Protection Agency), en particulier une composition dépourvue d'éthanol, et transparente. On entend ici par composition parfumante, une composition peu visqueuse dans laquelle un ou plusieurs ingrédients parfumants sont totalement solubilisés et qui, contrairement à une composition cosmétique, n'a pas pour but de pénétrer la peau lors de son application. L'invention a donc pour objet une composition parfumante sous forme d'une émulsion huile-dans-eau ou eau-dans-huile, l'huile contenant au moins un ingrédient parfumant, ladite émulsion comprenant de 5 à 50% en poids d'une phase dispersée et de 95 à 50% en poids d'une phase continue, la différence entre les indices de réfraction n de ladite phase dispersée et de ladite phase continue étant inférieure ou égale à environ 0,003, de préférence inférieure ou égale à environ 0,001.

La viscosité de la composition parfumante selon l'invention est inférieure à 10 Pa.s, indépendemment de la nature de l'émulsion.

Dans le cas d'une émulsion huile-dans-eau, la phase continue est formée par l'eau (phase aqueuse) et la phase dispersée est formée par l'huile (phase huileuse). A l'inverse, s'il s'agit d'une émulsion eau-dans-huile, aussi connue sous le nom d'émulsion inverse, la phase continue est formée par l'huile dans laquelle l'eau est dispersée, formant ainsi la phase dispersée.

Les ingrédients parfumants se trouvent dissous dans l'huile, en raison de leur caractère hydrophobe.

La composition parfumante se présente sous forme d'un parfum, d'une eau de toilette ou d'une eau de Cologne.

Les compositions parfumantes de l'invention sont obtenues en ajoutant à une émulsion certains ingrédients qui agissent sur les deux phases qui la constituent.

Les ingrédients que l'on ajoute à la composition de l'invention et qui sont spécifiés ci-après ont pour effet de modifier l'indice de réfraction des deux phases, de façon à former une émulsion transparente. Nous avons en effet pu constater que les émulsions transparentes de l'invention peuvent être obtenues lorsque la différence entre les indices de réfraction de la phase dispersée et de la phase continue ne dépasse pas environ 0,003. De préférence, cette différence est inférieure ou égale à environ 0,001.

La transparence des émulsions de l'invention est caractérisée par une transmission mesurée à 600 nm (cellule de 1 cm d'épaisseur), typiquement supérieure à 60% et pour beaucoup d'émulsions, supérieure à 80%.

Il convient de noter ici que l'indice de réfraction n de l'eau est de 1,33, et que les compositions parfumantes typiquement utilisées présentent un indice n généralement compris entre 1,45 et 1,55. L'adjonction de certains ingrédients permet de rapprocher les indices de réfraction respectifs de chacune des 2 phases jusqu'à ce que leur différence soit dans la plage définie ci-dessus.

Nous avons constaté que, dans le contexte de l'invention, on peut utiliser un certain nombre d'agents capables d'agir sur les deux phases de façon souhaitée et dont le choix dépend de critères individuels pouvant être établis pour chaque composition. Les agents susmentionnés seront ainsi sélectionnés en fonction de leur compatibilité avec la nature du parfum, leur compatibilité avec la peau, le toucher désiré du parfum transparent après application sur la peau, et l'inertie chimique de ces agents vis-à-vis des ingrédients parfumants. En outre, l'agent qui sera utilisé dans le but d'agir sur la phase aqueuse doit bien entendu être soluble dans celle-ci, de même que l'agent utilisé pour agir sur la phase huileuse doit être soluble dans l'huile.

On peut alors utiliser n'importe quel agent qui répond à un ou plusieurs des critères importants pour une composition parfumante donnée, dans la mesure où cet agent est capable de rehausser l'indice de réfraction n de la phase aqueuse et de baisser l'indice de réfraction n de la phase huileuse, de façon à ce que la différence entre ces deux indices de réfraction soit comprise dans la gamme mentionnée auparavant, à savoir qu'elle soit inférieure ou égale à environ 0,003.

L'agent utilisé peut aussi bien être liquide que solide.

Lorsque l'agent est liquide, nous avons utilisé, à titre préférentiel, un agent dont l'indice de réfraction n est supérieur ou égal à environ 1,40, les valeurs de n les plus appropriées étant celles supérieures ou égales à environ 1,43, dans le cas où ledit agent agit sur la phase aqueuse. En revanche, si l'agent agit sur la phase huileuse, son indice de réfraction n sera de préférence inférieur ou égal à environ 1,43, un indice n inférieur ou égal à environ 1,40 étant préféré.

Si l'on choisit d'ajouter un agent solide à une composition parfumante de l'invention, il est clair que ledit solide doit se dissoudre dans la phase huileuse ou la phase aqueuse de façon à baisser, respectivement rehausser, l'indice de réfraction de la phase sur laquelle il agira. Dans le contexte de la présente invention, le choix de cet agent solide sera alors aussi fonction de sa capacité à se dissoudre complètemenet dans la phase sur laquelle il agira et à changer de façon appropriée l'indice de réfraction de celle-ci.

Selon la présente invention, on préfère utiliser, en tant qu'agent agissant sur la phase aqueuse, au moins un composé choisi parmi l'urée, un diol, un triol ou un polyol de nature mono-, poly- ou oligomérique, ou des dérivés de ceux-ci. Des exemples de diols ou triols monomériques incluent l'éthylèneglycol, le 1,2-propanediol, le 1,3-propanediol, le 1,2-butanediol, le 1,3-butanediol, le 1,4-butanediol, le 2,3-butanediol, le 2-méthyl-1,3-propanediol, le 1,6-hexanediol, le néopentylglycol, le triméthylol-propane et le glycérol. Des exemples de diols, triols ou polyols oligo- ou polymériques incluent le diéthylèneglycol, le triéthylèneglycol, le dipropylèneglycol, les polyéthylèneglycols ayant une longueur de chaîne variée. Parmi les dérivés des alcools di-, tri- ou polyfonctionnels, on peut citer par exemple le produit commercialisé sous le nom de Lubrajel® (origine : United Guardian Inc., Hauppauge, NY, USA, distribué par Sederma, Le Perrey en Yvelines, France) contenant des polyméthacrylates de glycéryle stabilisés avec du propylène glycol.

En tant qu'agent agissant sur la phase huileuse, c'est-à-dire sur la phase dans laquelle se trouvent les ingrédients parfumants d'une composition donnée, on préfère ajouter un fluide silicone volatile. On entend ici par fluide silicone volatile, un fluide ayant une tension de vapeur à température ambiante supérieure ou égale à celle de l'éthanol. Des exemples non limitatifs de fluides silicone pouvant être utilisés selon la présente invention incluent des polydiméthylsiloxanes linéaires ou cycliques ayant 2 à 10 atomes de silicium. de préférence de 4 à 6 atomes de silicium, tels que, par exemple, le décaméthyl tétrasiloxane, l'octaméthylcyclotétrasiloxane, le décaméthylcyclopenta-siloxane ou le dodécaméthyl-cyclohexasiloxane, ou tout mélange de ces composés. Des silicones de ce type portent des désignations CTFA telles que diméthicone (en cas de siloxanes linéaires) et cyclométhicone (en cas de siloxanes cycliques). Ces fluides sont commercialisés par de nombreuses entreprises, par exemple : Dow Coming : Fluids DC 244, 245, 246, 344 EU, 345 EU (mélange de cyclométhicones) ou Fluid DC 200 (hexaméthyldisiloxane); General Electric Silicones : SF 1173, 1202, 1204 (mélange de cyclométhicones), SF 1214 (mélange de cyclométhicones et diméthicones) ou SF 96 (mélange de diméthicones) ; Wacker Silicones : Siloxane F 222, 223, 250, 251 (mélange de cyclométhicones), Siloxane F 221, Silicone Fluid SF 96, SWS 101 (mélange de diméthicones) ; Chemische Fabrik Th. Goldschmidt AG : Abil® K4, B 8839 (mélange de cyclométhicones), Abil® 10-10000 (mélange de diméthicones) ; Witco : L 7087 (diméthicone copolyol méthyl éther), L 7607 (diméthicone copolyol).

Les meilleurs résultats ont été obtenus avec le Fluid DC 345 EU, qui est un mélange composé d'environ 3 parties de décaméthylcyclopentasiloxane, environ 1 partie de docécaméthylcyclohexasiloxane et de faibles quantités d'octaméthylcyclotétrasiloxane.

Les silicones cités auparavant, bien qu'étant volatiles, ne font pas partie des substances classées dans le groupe des solvants organiques volatiles (VOC selon l'E.P.A.) et sont aussi de ce fait avantageusement utilisés dans la présente invention.

D'autres substances ajoutées à la phase huileuse, ont donné de bons résultats. On peut citer notamment des paraffines légères, telles que l'heptane, ou encore les fractions d'isoparaffine commercialisées sous le nom de Isopar®, par exemple Isopar® C ou Isopar® M par la compagnie Esso.

Une autre classe de substances que l'on peut utiliser avantageusement selon l'invention est constituée par des paraffines fluorées et perfluorées de nature oligomérique et polymérique qui sont d'un usage courant.

Il est clair que l'on peut également ajouter à la composition une moindre quantité d'un alcool inférieur comme l'éthanol ou l'isopropanol à la phase huileuse, c'est-à-dire au parfum. ou en utilisant un parfum qui est au moins partiellement dissous dans un alcool inférieur, ce qui permet également d'obtenir une émulsion transparente telle que souhaitée selon la présente invention. Cependant, les substances citées étant classées comme solvants organiques volatiles, leur utilisation est moins avantageuse.

L'indice de réfraction n d'une composition parfumante finie selon l'invention a une valeur comprise entre 1,40 et 1,44 environ.

Les émulsions de l'invention peuvent contenir de 5 à 50% en poids, de préférence de 10 à 35% en poids, de phase dispersée et de 95 à 50% en poids, de préférence de 90 à 65% en poids, de phase continue. Ces valeurs sont relatives au poids total de l'émulsion, et sont indépendantes du fait que les compositions de l'invention se trouvent sous forme d'émulsions huile-dans-eau ou eau-dans-huile.

La phase huileuse, quant à elle, comprend de 15 à 60%, de préférence de 20 à 50% de son poids en ingrédients parfumants.

La phase aqueuse comprend de 20 à 65% de son poids en eau.

Les émulsions de l'invention contenant les ingrédients spécifiés plus haut peuvent présenter telles quelles une stabilité suffisante lors du stockage. C'est le cas en particulier lorsqu'un ou plusieurs des agents qui sont ajoutés dans le but d'agir sur l'indice de réfraction de l'une ou l'autre phase sont capables de stabiliser l'émulsion en raison de leurs propriétés tensioactives.

Le cas échéant, on ajoute encore à la composition de l'invention au moins un agent tensioactif approprié afin d'obtenir une émulsion stable. Cet agent tensioactif sera utilisé dans des proportions allant de 0 à 8%, de préférence de 0,1 à 5% en poids, par rapport au poids total de l'émulsion. Les meilleurs résultats ont été obtenus en utilisant de 2 à 5% en poids d'agent tensioactif.

Des agents tensioactifs de nature variée peuvent être utilisés dans le contexte de l'invention. On peut citer les agents tensioactifs non-ioniques, cationiques, anioniques, amphotériques et les phospholipides, qui se prêtent tous à une utilisation selon la présente invention. On utilise de préférence un agent tensioactif non ionique ou un mélange de 2 tensioactifs non ioniques. En tant qu'exemple non limitatif, on peut citer le nonylphénol éthoxylé comportant 9 à 10 unités d'éthylène glycol (en vente sous le nom de Triton® N-101 ; origine : Fluka, Suisse) ou l'undécanol éthoxylé comportant 8 unités d'éthylène glycol (en vente sous le nom de Imbentin® 080 ; origine : Kolb AG, Suisse). D'autres exemples incluent les tensioactifs connus sous le nom de Tween ® (origine : ICI, Angleterre), tels que Tween ® 20 [polyoxyéthylène (20) sorbitan monolaurate]. Tween ® 40 [polyoxy-éthylène (20) sorbitan monopalmitate], Tween ® 60 [polyoxyéthylène (20) sorbitan monostéarate] et le Tween ® 80 [polyoxyéthylène (20) sorbitan monooléate], et les tensioactifs vendus sous le nom de Span ® (origine : ICI, Angleterre), dont il convient de citer le Span ® 20 (sorbitan monolaurate), le Span ® 40 (sorbitan monopalmitate), le Span ® 60 (sorbitan monostéarate) et le Span ® 80 (sorbitan monooléate). On peut encore citer le Cremophor ® RH40 et RH60 (origine : BASF AG, Allemagne, qui sont des huiles de ricin hydrogénées et éthoxylées), le Genapol ® (origine : Hoechst AG, Allemagne, un (alcool polyglycol éther) laurylsulfate de sodium), le tensioactif connu sous le nom de Poloxamer ® 407 (un copolymère dibloc d'oxyde d'éthylène et d'oxyde de propylène, aussi vendu sous les noms de Pluronic ® F127 et Pluracare ® F127, origine : BASF AG, Allemagne); le Tetronic ® (origine: BASF AG, Allemagne); le DC 3225 C, le DC 5200, le DC 193 (origine: Dow Corning, USA); Abil ® Em 97 (origine: Goldschmidt).

Les ingrédients parfumants pouvant être utilisés selon l'invention sont tous des ingrédients d'usage courant dans la parfumerie. Leur nature n'appelle pas une description plus détaillée ici, qui ne saurait d'ailleurs être exhaustive, l'homme de l'art étant à même de les choisir de par ses connaissances générales et en fonction de l'effet olfactif recherché. Ces ingrédients parfumants appartiennent à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres ouvrages de nature similaire.

Les émulsions de l'invention peuvent être aisément préparées par des méthodes de mélange et homogénéisation conventionnelles qui n'appellent pas ici une description plus détaillée.

Selon la présente invention, on crée des émulsions ayant une taille moyenne de goutte supérieure à 200 nm.

L'invention sera maintenant illustrée par les exemples suivants non limitatifs, dans lesquels les températures sont indiquées en degrés Celsius, les proportions des composés sont données en % en poids, et les abréviations ont le sens usuel dans l'art.

## Manières de réaliser l'invention

### Exemples 1 et 2

Préparation de compositions parfumantes transparentes sous forme d'émulsions huile-dans-eau

Les émulsions de type huile-dans-eau contenant des bases parfumantes ont été préparées à partir des ingrédients spécifiés ci-dessous, par des méthodes courantes dans l'art.

**Exemple 1**

| Ingrédients | Parties en poids |
|---|---|
| Base parfumante * | 10,05 |
| Silicone DC ⌇ 345 [1] | 24,93 |
| Perfluorodécaline | 2,23 |
| Eau (pH 7) | 21,35 |
| 1,2-Butanediol | 36,43 |
| Tetronic ® 704 [2] | 2,50 |
| Abil ® Em 97 [3] | 2,50 |
| Total | 100,00 |

1) origine : Dow Corning

2) origine : BASF AG

3) origine : Goldschmidt

\* La base parfumante a été obtenue par mélange des ingrédients suivants :

| Ingrédients | Parties en poids |
|---|---|
| Acétate de citronellyle | 3 |

| | |
|---|---:|
| Acétate de géranyle | 9 |
| Acétate de linalyle | 276 |
| Aldéhyde C10 à 10% * | 3 |
| Aldehyde C12 à 10% * | 12 |
| Anthranilate de méthyle | 16 |
| Essence de bergamote | 226 |
| Cetalox [®1)] | 5 |
| Essence de citron | 318 |
| Dihydromyrcénol [2)] | 60 |
| Dipropylène glycol | 20 |
| Elemi [3)] à 10% * | 20 |
| Fleuria 41063 B [4)] | 3 |
| Ethyl linalol | 66 |
| 3-(4-Méthoxyphényl)-2-méthylpropanal [4)] à 10% * | 30 |
| Géraniol | 6 |
| Habanolide [ɛ 5)] à 50% * | 130 |
| Hedione [® 6)] | 215 |
| Hedione [®] HC [7)] | 72 |
| Indol à 10% ** | 12 |
| Iso E super [8)] | 85 |
| Essence de lavandin grosso | 26 |
| Liffarome [®9)] à 1% * | 20 |
| Linalol | 40 |
| Essence de mandarine sfuma | 5 |
| Essence de menthe crépue à 10% * | 30 |
| Essence de Néroli bigarade | 130 |
| Essence d'orange Portugal Floride | 80 |
| Phénéthylol | 9 |
| Essence de petitgrain | 63 |
| Pipol | 5 |
| Essence de romarin | 16 |

| | |
|---|---|
| Terpinéol | 9 |
| Essence de violette | 50 |
| Zestover [10] à 1% * | 30 |
| Total | 2100 |

\* dans le dipropylène glycol (DIPG)

\*\* dans la triéthanolamine

1) 8,12-époxy-13,14,15,16-tétranorlabdane

2) origine : International Flavors and Fragrances, USA

3) 5-allyl-1,2,3-trimethoxybenzène ; origine : Calchauvet, Grasse, France

4) origine : Firmenich SA, Genève, Suisse

5) pentadécénolide ; origine : Firmenich SA, Genève, Suisse

6) dihydrojasmonate de méthyle ; origine : Firmenich SA, Genève, Suisse

7) dihydrojasmonate de méthyle à haute teneur en isomère *cis* ; origine : Firmenich SA, Genève, Suisse

8) 1-(octahydro-2,3,8,8-tétraméthyl-2-naphtalényl)-1-éthanone ; origine : International Flavors and Fragrances, USA

9) carbonate de 3-hexenyle méthyle ; origine : International Flavors and Fragrances, USA

10) 9-décen-1-ol ; origine : International Flavors and Fragrances, USA

L'indice de réfraction de chacune des deux phases de la composition a été mesuré à température ambiante, une fois la phase aqueuse et la phase huileuse en contact et avant d'ajouter l'agent tensioactif. L'indice de réfraction n de la phase aqueuse était de 1,4098 et celui de la phase huileuse de 1,4112, donnant ainsi une émulsion transparente. La transmission de l'émulsion, mesurée à une longeur d'onde de 600 nm dans une cellule de 1 cm d'épaisseur, est de 91,5%.

**Exemple 2**

| Ingrédients | Parties en poids |
|---|---|
| Base parfumante * | 10,29 |
| Silicone DC *345 [1] | 23,22 |

| | |
|---|---|
| Perfluorodécaline | 2,14 |
| Eau (pH 7) | 22,29 |
| 1,2-Butanediol | 38,06 |
| DC 193 [2] | 2,00 |
| DC 3225C [2] | 2,00 |
| Total | 100,00 |

1) voir exemple 1

2) origine : Dow Corning

*     La base parfumante a été obtenue par mélange des ingrédients suivants :

| Ingrédients | Parties en poids |
|---|---|
| Acétate de benzyle | 250 |
| Acétate de pipol | 70 |
| Acétate de styrallyle | 230 |
| Aldéhyde phénylacétique | 10 |
| Ambrettolide ® [1] | 10 |
| Astrotone | 300 |
| Essence de bergamote | 1160 |
| β-Ionone | 550 |
| Essence de cassis | 150 |
| Cetalox ® [2] à 50% * | 60 |
| Essence de citron | 850 |
| Citronellol | 210 |
| Damascénone | 20 |
| 4-Décanolide | 20 |
| Dihydromyrcénol [3] | 440 |
| Dipropylène glycol | 20 |
| Ethyl linalol | 720 |
| 7-Méthyl-2H.4H-1,5-benzodioxépin-3-one [4] | 100 |
| Floralozone ® [5] | 50 |

| | |
|---|---|
| 3-(4-Méthoxyphényl)-2-méthylpropanal [4] | 170 |
| Fructone [®6] | 100 |
| Galbex [® 4] | 50 |
| γ-Damascone | 5 |
| Essence de géranium | 30 |
| Essence de pamplemousse | 100 |
| Habanolide [® 7] | 1120 |
| Hedione [® 8] | 2890 |
| Hedione [® HC 9] | 950 |
| Heliopropanal [10] | 400 |
| Indol | 35 |
| Iso E Super [11] | 380 |
| Essence de lavandin grosso | 40 |
| Liffarome [® 12] | 1 |
| Lilial [® 13] | 1050 |
| Lyral [® 14] | 430 |
| Essence de mandarine sfuma | 270 |
| Melonal [15] | 3 |
| Essence de menthe crépue | 20 |
| Peony 434017 [4] | 60 |
| Peony white HS 100001 [4] | 200 |
| Phénéthylol | 80 |
| Phénylhexanol | 50 |
| Pipol | 20 |
| Essence d'orange | 500 |
| Rosalva [16] | 4 |
| Salicylate de benzyle | 400 |
| Salicylate de pipol | 400 |
| BHT [17] à 10% ** | 200 |
| Zestover [18] | 22 |
| Total | 15200 |

\*    dans le 2-(2-éthoxyéthoxy)-1-éthanol

\*\*   dans le dipropylène glycol

1)   origine : Givaudan-Roure SA, Vernier, Suisse

2)   8,12-époxy-13,14,15,16-tétranorlabdane ; origine : Firmenich SA, Genève, Suisse

3)   origine : International Flavors and Fragrances Inc., USA

4)   origine : Firmenich SA, Genève, Suisse

5)   3-(4-éthylphényl)-2,2-diméthylpropanal + 3-(2-éthylphényl)-2,2-diméthylpropanal ; origine : International Flavors and Fragrances Inc., USA

6)   2-méthyl-1,3-dioxalane-2-acétate d'éthyle ; origine : International Flavors and Fragrances Inc., USA

7)   pentadécénolide ; origine : Firmenich SA, Genève, Suisse

8)   dihydrojasmonate de méthyle ; origine : Firmenich SA, Genève, Suisse

9)   dihydrojasmonate de méthyle à haute teneur en isomère cis ; origine : Firmenich SA, Genève, Suisse

10)  3-(1,3-benzodioxol-5-yl)-2-méthylpropanal ; origine : Firmenich SA, Genève, Suisse

11)  1-(octahydro-2,3,8.8-tétraméthyl-2-naphtalényl)-1-éthanone ; origine : International Flavors and Fragrances Inc., USA

12)  carbonate de 3-hexényle-méthyle ; origine : International Flavors and Fragrances Inc., USA

13)  3-(4-tert-butylphényl)-2-méthylpropanal ; origine : Givaudan-Roure SA, Vernier, Suisse

14)  4-(4-hydroxy-4-méthylpentyl)-3-cyclohexène-1-carbaldéhyde + 3-(4-hydroxy-4-méthylpentyl)-3-cyclohexène-1-carbaldéhyde ; origine : International Flavors and Fragrances Inc., USA

15)  2,6-diméthyl-5-heptanal ; origine : Givaudan-Roure SA, Vernier, Suisse

16)  9-decen-1-ol ; origine : International Flavors and Fragrances Inc., USA

17)  2,6-di-tert-butyl-4-hydroxytoluène

18)  2,4-diméthyl-3-cyclohexene-1-carbaldehyde ;  origine :  Firmenich SA,  Genève, Suisse

L'indice de réfraction de chacune des deux phases a été mesuré à température ambiante, une fois la phase aqueuse et la phase huileuse en contact et avant d'ajouter l'agent tensioactif. L'indice de réfraction n de la phase aqueuse était de 1,4109 et celui de la phase huileuse de 1,4132, donnant ainsi une émulsion transparente. La transmission de l'émulsion, mesurée dans les mêmes conditions que dans l'Exemple 1, est de 98,2%.

Exemples 3 et 4

Préparation de compositions parfumantes transparentes sous forme d'émulsions eau-dans-huile

Les émulsions de type eau-dans-huile contenant des bases parfumantes ont été préparées à partir des ingrédients spécifiés ci-dessous, par des méthodes courantes dans l'art.

**Exemple 3**

| Ingrédients | Parties en poids |
|---|---|
| Base parfumante * | 10,20 |
| Silicone DC ® 345 [1] | 55,12 |
| Perfluorodécaline | 4,17 |
| Eau (pH 7) | 11,55 |
| 1,2-Butanediol | 16,96 |
| Abil ® EM 97 [2] | 2,00 |
| Total | 100,00 |

1) voir exemple 1

2) origine : Glodschmidt

* voir exemple 1

L'indice de réfraction de chacune des deux phases a été mesuré à température ambiante, une fois la phase aqueuse et la phase huileuse en contact et avant d'ajouter l'agent tensioactif. L'indice de réfraction n de la phase aqueuse était de 1,4050 et celui de la phase huileuse de 1,4070, donnant ainsi une émulsion transparente. La transmission de l'émulsion, mesurée dans les mêmes conditions que dans l'Exemple 1, est de 65,4%.

**Exemple 4**

| Ingrédients | Parties en poids |
|---|---|
| Base parfumante * | 10,20 |
| Silicone DC ® 345 [1] | 55,12 |
| Perfluorodécaline | 4,17 |
| Eau (pH 7) | 11,55 |
| 1,2-Butanediol | 16,96 |
| Abil ® Em 97 [2] | 2,00 |
| Total | 100,00 |

1) voir exemple 1

2) voir exemple 3

* voir exemple 2

L'indice de réfraction de chacune des deux phases a été mesuré à température ambiante, une fois la phase aqueuse et la phase huileuse en contact et avant d'ajouter l'agent tensioactif. L'indice de réfraction n de la phase aqueuse était de 1,4075 et celui de la phase huileuse de 1,4068, donnant ainsi une émulsion transparente. La transmission de l'émulsion, mesurée dans les mêmes conditions que dans l'Exemple 1, est de 58,8%.
En mélangeant les ingrédients spécifiés ci-dessus, on a obtenu une composition parfumante sous forme d'une émulsion du type eau-dans-huile stable.

**Revendications**

1. Parfum, eau de toilette ou eau de Cologne sous forme d'une émulsion huile-dans-eau ou eau-dans-huile, l'huile contenant au moins un ingrédient parfumant, ladite composition comprenant de 5 à 50% en poids d'une phase dispersée et de 95 à 50% en poids d'une phase continue, la différence entre les indices de réfraction n de ladite phase dispersée et de ladite phase continue étant inférieure ou égale à 0,003, de préférence inférieure ou égale à 0,001 et la viscosité de l'émulsion étant inférieure à 10 Pa.s.

2. Parfum, eau de toilette ou eau de Cologne selon la revendication 1 **caractérisés en ce que** la différence entre les indices de réfraction est atteinte par l'action d'un agent susceptible de changer l'indice de réfraction de l'une ou l'autre desdites phases.

3. Parfum, eau de toilette ou eau de Cologne selon la revendication 1 ou 2, **caractérisés en ce que** l'agent agissant sur la phase huileuse est un agent liquide ayant un indice de réfraction n inférieur ou égal à environ 1,43 et l'agent agissant sur la phase aqueuse est un agent liquide ayant un indice de réfraction n supérieur ou égal à environ 1,40.

4. Parfum, eau de toilette ou eau de Cologne selon la revendication 3, **caractérisés en ce que** l'indice de réfraction de l'agent liquide agissant sur la phase huileuse est inférieur ou égal à environ 1,40 et l'indice de réfraction de l'agent liquide agissant sur la phase aqueuse est supérieur ou égal à environ 1,43.

5. Parfum, eau de toilette ou eau de Cologne selon l'une des revendications 1 à 4, **caractérisés en ce que** ledit agent agissant sur la phase huileuse est choisi parmi les silicones volatiles ayant une tension de vapeur supérieure ou égale à celle de l'éthanol, une paraffine ou une paraffine fluorée ou perfluorée.

6. Parfum, eau de toilette ou eau de Cologne selon la revendication 5, **caractérisés en ce que** ladite silicone volatile est un polydiméthylsiloxane linéaire ou cyclique ayant 2 à 10 atomes de silicium.

7. Parfum, eau de toilette ou eau de Cologne selon la revendication 5, **caractérisés en ce que** ladite silicone est l'octaméthylcyclotétrasiloxane, le décaméthylcyclopenta-siloxane ou le dodécaméthylcyclohexasiloxane.

8. Parfum, eau de toilette ou eau de Cologne selon l'une des revendications 1 à 7, **caractérisés en ce que** ledit agent agissant sur la phase aqueuse est choisi parmi les diols, triols ou polyols de nature mono-, oligo- ou polymérique ou un dérivé de ceux-ci, ou l'urée.

9. Parfum, eau de toilette ou eau de Cologne selon la revendication 8, **caractérisés en ce que** ledit diol, triol ou polyol est l'éthylèneglycol, le 1,2-propanediol, le 1,3-propanediol, le 1,2-butanediol, le 1,3-butanediol, le 1,4-butanediol, le 2,3-butanediol, le 2-méthyl-1,3-propanediol, le 1,6-hexanediol, le néopentylglycol, le triméthylolpropane, le glycérol, le diéthylèneglycol, le triéthylèneglycol, le dipropylèneglycol, un polyéthylèneglycol ayant une longueur de chaîne variée, ou un polyméthacrylate de glycéryle.

10. Parfum, eau de toilette ou eau de Cologne selon la revendication 9, **caractérisés en ce que** le diol est le 1,2-butanediol.

11. Parfum, eau de toilette ou eau de Cologne selon l'une des revendications 1 à 10, **caractérisés en ce qu'**ils comprennent de 0 à 8% en poids d'un agent tensioactif.

12. Parfum, eau de toilette ou eau de Cologne selon la revendication 11, **caractérisés en ce que** l'agent tensioactif est présent dans une quantité de 0,1 à 5% en poids.

13. Parfum, eau de toilette ou eau de Cologne selon la revendication 11 ou 12, **caractérisés en ce que** ledit agent tensioactif est non-ionique.

**Patentansprüche**

1. Parfum, Eau-de-Toilette oder Eau de Cologne in Form einer Öl-in-Wasser- oder Wasser-in-Öl-Emulsion, wobei das Öl mindestens einen Duftstoff enthält und die Zusammensetzung 5 bis 50 Gew.-% einer dispergierten Phase und 95 bis 50 Gew.-% einer kontinuierlichen Phase umfaßt, wobei der Unterschied zwischen den Brechungsindices n der dispergierten Phase und der kontinuierlichen Phase weniger als oder gleich 0,003, bevorzugt weniger als oder gleich 0,001 ist und die Viskosität der Emulsion weniger als 10 Pa.s beträgt.

2. Parfum, Eau-de-Toilette oder Eau de Cologne nach Anspruch 1, **dadurch gekennzeichnet, daß** der Unterschied zwischen den Brechungsindices durch die Einwirkung eines Mittels erzielt wird, das imstande ist, den Brechungsindex der einen oder der anderen dieser Phasen zu ändern.

3. Parfum, Eau-de-Toilette oder Eau de Cologne nach Anspruch 1 oder 2, **dadurch gekennzeichnet, daß** das auf die Ölphase einwirkende Mittel ein flüssiges Mittel mit einem Brechungsindex n von weniger als oder gleich etwa 1,43 ist, und das auf die wäßrige Phase einwirkende Mittel ein flüssiges Mittel mit einem Brechungsindex n von mehr als oder gleich etwa 1,40 ist.

4. Parfum, Eau-de-Toilette oder Eau de Cologne nach Anspruch 3, **dadurch gekennzeichnet, daß** der Brechungsindex des auf die Ölphase einwirkenden flüssigen Mittels weniger als oder gleich etwa 1,40 ist und der Brechungsindex des auf die wäßrige Phase einwirkenden flüssigen Mittels mehr als oder gleich etwa 1,43 ist.

5. Parfum, Eau-de-Toilette oder Eau de Cologne nach einem der Ansprüche 1 bis 4, **dadurch gekennzeichnet, daß** das auf die Ölphase einwirkende Mittel ausgewählt ist aus flüchtigen Siliconen mit einem Dampfdruck, der höher als der oder gleich dem von Ethanol ist, einem Paraffin, oder einem fluorierten oder perfluorierten Paraffin.

6. Parfum, Eau-de-Toilette oder Eau de Cologne nach Anspruch 5, **dadurch gekennzeichnet, daß** das flüchtige Silicon ein geradkettiges oder cyclisches Polydimethylsiloxan mit 2 bis 10 Siliciumatomen ist.

7. Parfum, Eau-de-Toilette oder Eau de Cologne nach Anspruch 5, **dadurch gekennzeichnet, daß** das Silicon Octamethylcyclotetrasiloxan, Decamethylcyclopentasiloxan oder Dodecamethylcyclohexasiloxan ist.

8. Parfum, Eau-de-Toilette oder Eau de Cologne nach einem der Ansprüche 1 bis 7, **dadurch gekennzeichnet, daß** das auf die wäßrige Phase einwirkende Mittel aus mono-, oligo- oder polymeren Diolen, Triolen oder Polyolen oder einem Derivat von diesen oder Harnstoff ausgewählt ist.

9. Parfum, Eau-de-Toilette oder Eau de Cologne nach Anspruch 8, **dadurch gekennzeichnet, daß** das Diol, Triol oder Polyol Ethylenglycol, 1,2-Propandiol, 1,3-Propandiol, 1,2-Butandiol, 1,3-Butandiol, 1,4-Butandiol, 2,3-Butandiol, 2-Methyl-1,3-propandiol, 1,6-Hexandiol, Neopentylglycol, Trimethylolpropan, Glycerin, Diethylenglycol, Triethylenglycol, Dipropylenglycol, ein Polyethylenglycol unterschiedlicher Kettenlänge, oder ein Glycerylpolymethacrylat ist.

10. Parfum, Eau-de-Toilette oder Eau de Cologne nach Anspruch 9, **dadurch gekennzeichnet, daß** das Diol 1,2-Butandiol ist.

11. Parfum, Eau-de-Toilette oder Eau de Cologne nach einem der Ansprüche 1 bis 10, **dadurch gekennzeichnet, daß** sie 0 bis 8 Gew.-% eines Tensids aufweisen.

12. Parfum, Eau-de-Toilette oder Eau de Cologne nach Anspruch 11, **dadurch gekennzeichnet, daß** das Tensid in einer Menge von 0,1 bis 5 Gew.-% vorliegt.

13. Parfum, Eau-de-Toilette oder Eau de Cologne nach Anspruch 11 oder 12, **dadurch gekennzeichnet, daß** das Tensid nicht-ionisch ist.

**Claims**

1. Perfume, toilet water or Cologne in the form of an oil-in-water or water-in-oil emulsion, the oil containing at least one perfuming ingredient, said composition comprising from 5 to 50% by weight of a dispersed phase and from 95 to 50% by weight of a continuous phase, the difference between the refractive indices n of said dispersed phase and said continuous phase being less than or equal to 0.003, preferably less than or equal to 0.001, and the viscosity of the emulsion being less than 10 Pa.s.

2. Perfume, toilet water or Cologne according to claim 1, **characterised in that** the difference between the refractive indices is obtained by the action of an agent able to change the refractive index of one or the other of the two phases.

3. Perfume, toilet water or Cologne according to claim 1 or 2, **characterised in that** the agent acting on the oily phase is a liquid agent having a refractive index n of less than or equal to ca. 1.43 and the agent acting on the aqueous phase is a liquid agent having a refractive index n above or equal to ca. 1.40.

4. Perfume, toilet water or Cologne according to claim 3, **characterised in that** the refractive index of the liquid agent acting on the oily phase is less than or equal to ca. 1.40 and the refractive index of the liquid substance acting on the aqueous phase is above or equal to ca. 1.43.

5. Perfume, toilet water or Cologne according to any one of claims 1 to 4, **characterised in that** the agent acting on the oily phase is selected among the volatile silicones having a vapour pressure superior or equal to that of ethanol, a paraffin or a fluorinated or perfluorinated paraffin.

6. Perfume, toilet water or Cologne according to claim 5, **characterised in that** said volatile silicone is a linear or cyclic polydimethylsiloxane having from 2 to 10 silicon atoms.

7. Perfume, toilet water or Cologne according to claim 5, **characterised in that** said silicone is octamethylcyclotetra-siloxane, decamethylcyclopenta-siloxane or dodecamethylcyclohexa-siloxane.

8. Perfume, toilet water or Cologne according to any one of claims 1 to 7, **characterised in that** the aforementioned agent acting on the aqueous phase is selected among the diols, triols or polyols of mono-, oligo- or polymeric nature, or a derivative thereof, or urea.

9. Perfume, toilet water or Cologne according to claim 8, **characterised in that** said diol, triol, or polyol is ethyleneglycol, 1,2-propanediol, 1,3-propanediol, 1,2-butanediol, 1,3-butanediol, 1,4-butanediol, 2,3-butanediol, 2-methyl-1,3-propanediol, 1,6-hexanediol, neopentylglycol, trimethylolpropane, glycerol, diethyleneglycol, triethyleneglycol, dipropyleneglycol, a polyethyleneglycol having a varied chain length, or a glyceryl polymethacrylate.

10. Perfume, toilet water or Cologne according to claim 9, **characterised in that** the diol is 1,2-butanediol.

11. Perfume, toilet water or Cologne according to any one of claims 1 to 10, **characterised in that** it comprises from 0 and 8% by weight of a surfactant.

12. Perfume, toilet water or Cologne according to claim 11, **characterised in that** the surfactant is present in a quantity comprised between 0.1 and 5% by weight.

13. Perfume, toilet water or Cologne according to claim 11 or 12, **characterised in that** said surfactant is non-ionic.